# EUROPEAN PATENT SPECIFICATION

(11) **EP 2 666 500 B1**
(45) Date of publication and mention of the grant of the patent: **07.09.2016**
(21) Application number: 13169227.9
(22) Date of filing: 24.05.2013
(51) Int. Cl.: A61M 15/00

(54) **Inhaler device with an improved blister advancement mechanism**
Inhalatorvorrichtung mit verbessertem Blistervorrückmechanismus
Dispositif inhalateur avec mécanisme d'avancement de blister amélioré

(30) Priority: 25.05.2012 TR 201206167; 08.02.2013 TR 201301562; 15.02.2013 TR 201301847; 19.02.2013 TR 201301950
(43) Date of publication of application: 27.11.2013
(73) Proprietor: Arven Ilac Sanayi Ve Ticaret A.S., Istanbul 34460 (TR)
(72) Inventor: Toksöz, Zafer, 34460 Istanbul (TR); Cifter, Ümit, 34460 Istanbul (TR); Türkyilmaz, Ali, 34460 Istanbul (TR); Mutlu, Onur, 34460 Istanbul (TR)
(74) Representative: Sevinç, Erkan

(56) References cited:
- WO-A1-01/66061
- WO-A1-2010/114506
- US-A- 4 733 797
- US-A- 5 590 645
- US-A1- 2002 032 409
- US-A1- 2004 050 385
- US-A1- 2007 215 149
- US-A1- 2009 283 095

## Description

### Field of Invention

The present invention relates to a device for administering dry powder inhalation drugs.

The present invention particularly relates to improvements made in the blister advancement mechanism of dry powder inhaler devices.

### Prior Art

Diseases such as asthma, bronchitis, and COLD (Chronic Obstructive Lung Disease) substantially decrease the quality of human life, despite the developments which have been made in the diagnosis and therapy thereof in the recent years. It has been proposed to administer medicaments via inhalers for optimizing the treatment of such diseases. The inhaler route of treatment is the most preferred one and it is expected to remain so, as the first option, in the future. The most important advantage of using medicaments via inhalation is based on providing a more efficient therapy by making use of lower amounts of medicaments, delivering higher concentrations of medicaments to the airways, and particularly decreasing the systemic side effects of the medicaments. The most important causes of the lack of a satisfactory control of patients albeit the presence of quite efficient treatments against respiratory tract diseases are stated to be as the noncompliance, arising from the inefficient use of inhalers and from inadequate compliance to the physician-recommended treatments.

There have been developed various inhalation devices for administering inhalation drugs nowadays. These devices are basically classified into two groups, i.e. metered dose inhalers and dry powder inhalers. These types of devices are structurally provided with basic components such as an actuator, counter, body, lid, lock, etc. Additionally, powder inhalation drugs are kept in carriers such as blisters, capsules, etc. Blisters are composed of two basic parts, a main layer provided with cavities carrying the drug, and a strippable protective layer.

Such devices comprise an outer body and a lid provided thereon, an inner body disposed in the outer body, a blister strip disposed in the inner body, a lower holder comprising this strip in a rolled manner, and a gear and gear set aligned in an interconnected and functionally-compatible manner to store the main layer comprising cavities after the blister strip is moved and separated into its layers. A lever or trigger mechanism is developed to actuate this mechanism.

To the mechanism providing the motion of blisters in inhaler devices which comprise multiple blisters are exerted a force by means of a trigger or a lever and the mechanism is actuated accordingly. However, various problems are encountered in using the mechanism. Some of these are related to the levers actuating the mechanism. Such levers bring about some drawbacks such as difficulty in use and an extra movable volume requirement on the exterior of the inhaler body. The linear/axial movement of the trigger mechanism providing a size advantage and slid into the inner part of the inhaler device is converted into a circular motion by means of a gear to which it is connected. In the applications WO2010114506 and WO2010114505, for instance, the linear motion performed by a trigger is converted into a circular motion by means of a gear. This trigger is slid into a slot provided in the device body to provide an linear/axial motion, such that the force resulting from this motion is transmitted to a blister advancement mechanism in the device by means of a gear with which the trigger is in engagement. However, the gears serving to transmit and translate this drive are overloaded. Additionally, using extra gears gives rise to losses in force transmission. Eliminating this loss, in turn, requires more force to be applied. Another drawback is that slackness do occur between the extra gear and the contact surfaces. In result, this slackness and the exertion of extra force brings about a more important problem, which causes the gears in the mechanism to break down or wear out.

The use of currently-available inhaler devices requires certain training and practice. The development of inhaler devices always occurs in the form of practical systems, providing the patients with improved convenience of use. Selecting the proper device for a respective patient is also an important issue. Many criteria, such as a patient's cognitive and physical efficiency, ease of use, safety and price, etc. are considered while a device is selected.

US 4 733 797 discloses a dosage sealing, monitoring and dispensing assembly including a mounting receptacle and one or more cartridges removably connected to the receptacle. Each cartridge has sealed therewithin a fresh, supply of dosage units carried in moisture impervious shells on a flexible, segmented strip.

WO 01/66061 discloses an apparatus to dose medicaments and control taking in the medicaments, which apparatus consists of a storage unit to store tablets or capsules, a dosing unit and a counter. The characteristic feature of the apparatus is that the medicaments are packed in a medicament tape and stored in the storage unit and cylinders furthering said medicament tape are attached to the storage unit and the said cylinders are mechanically coupled with a manual driving unit.

US 2004/050385 discloses an inhaler for delivery of a dry powder medicament, the inhaler comprising a breath sensor for sensing the breath of a patient; means for transporting the medicament to a delivery position; and electro-mechanical coupling means for actuating said transport means, wherein said coupling means is directly or indirectly responsive to the breath sensor.

US 2009/283095 A1 discloses an inhaler comprising: a body; at least one cover element that is movable between a closed position and an open position; individual reservoirs formed on a reservoir substrate; movable support means that receive said reservoir substrate and that are displaceable between a non-dispensing position and a dispensing position; and an indicator device for indicating doses that have been dispensed or that remain to be dispensed.

US 5 590 645 discloses an inhalation device is described for use with a medicament pack in which at least one container for medicament in powder form is defined between two sheets peelably secured to one another. The device comprises means for peeling the sheets apart at an opening station to open the container; and an outlet, communicating with the opened container, through which a user can inhale medicament in powder form from the opened container.

US 2002/032409 relates to a dispenser for dischargeable media, preferably containing at least one active pharmaceutical substance and packed in portioned manner in storage chambers of a storage means, as well as a conveying device for the supply of the storage means.

In result, there is a novelty required in the field of inhaler devices, providing high-accuracy operation, as well as advantages in terms of cost, size, and use.

### Objects and Brief Description of Invention

The present invention relates to an improved inhaler device for use with dry powder inhaling purposes, eliminating all aforesaid problems and bringing additional advantages to the relevant prior art.
Accordingly, the main object of the present invention is to provide a dry powder inhaler device, which can be operated at a desired accuracy, provide the peeling off of the blister to be used, and perform this process faultlessly as compared to precedent devices.

Another object of the present invention is to reduce the force which needs to be exerted to the trigger of the device to operate the same.

As a result of reducing the force exerted to operate the device, a further object of the present invention is to avoid any breaking and wearing-out of device components by decreasing the force applied to the surface of the gears and the mechanism thereof.

In order to achieve all objects described above and to emerge in the following detailed description, a blister advancement mechanism for a dry powder inhaler device comprising an outer body and a trigger disposed to axially displace in this outer body is developed, said blister advancement mechanism being structured to advance a blister strip having drug carrying cavities and to release the drug from the next drug carrying cavity of the blister strip and further comprising a transmission gear and a set of interconnected gears.

The invention is defined in the appended claim 1.

According to the present invention, the blister advancement mechanism comprises at least one actuator lever, which is coupled to the trigger on one side, and to the circular transmission gear on the other side, and is provided with teeth on at least a portion thereof, to transmit a force exerted to the trigger to the gear set and to convert the linear/axial motion of the trigger into circular motion.

According to the present invention, the actuator lever and the trigger comprise a groove and a pivot connection pin to enable the actuator lever to perform a rotational motion.

In a preferred embodiment according to the present invention, the teeth on the actuator lever are disposed on a circular surface.

In a preferred embodiment according to the present invention, the teeth on the actuator lever are disposed on a linear surface.

ccording to the present invention, the actuator lever and the trigger are fixed to each other to enable the actuator lever to perform an linear/axial motion.

Structural and characteristic features, and all advantages of the present invention shall be made clear by means of the accompanying figures described here below and a detailed description written by making references to said figures; therefore, the present invention must be evaluated by taking into consideration these figures and the detailed description as well.

### Brief Description of Figures

Figure 1 is a schematic view of a representative embodiment according to the present invention.

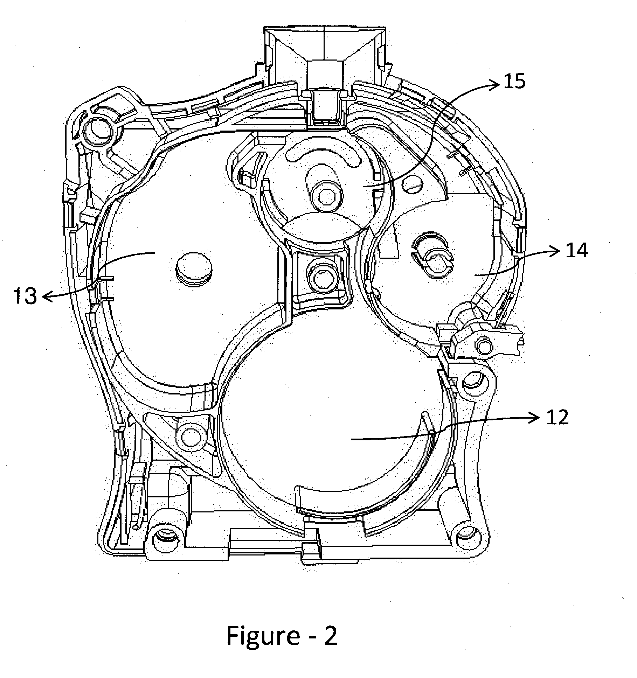

Figure 2 is a schematic view of a representative embodiment of an outer body and an inner body according to the present invention.
Figure 3 is a schematic view of a representative embodiment of the outer body, an inner body, and the mechanism according to the present invention.
Figure 4 is a schematic view of a representative embodiment of the outer body and the mechanism according to the present invention.

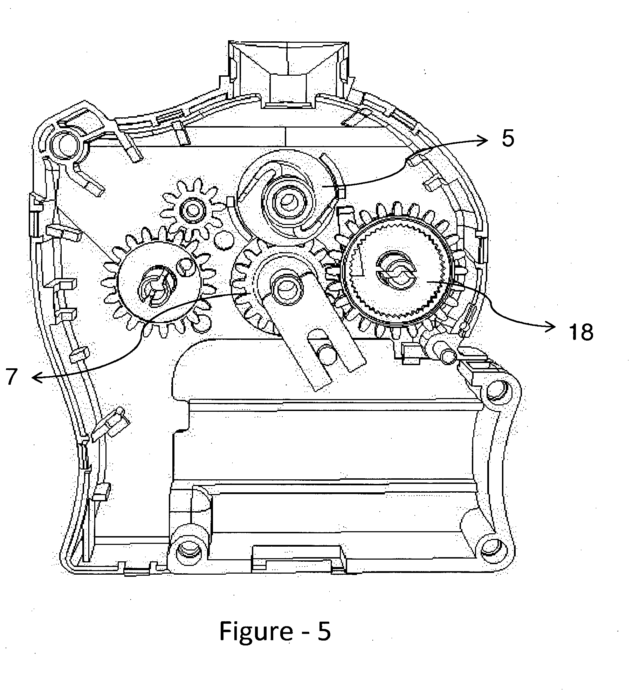

Figure 5 is a schematic view of a representative embodiment of the outer body and the mechanism according to the present invention.
Figure 6 is a schematic view of a representative embodiment of the mechanism according to the present invention.
Figure 7 is a schematic view of a representative embodiment of the mechanism according to the present invention.
Figure 8 is a schematic view of a representative embodiment of the inner body according to the present invention.
Figure 9a is a schematic view of a representative embodiment of a guide gear according to the present invention.
Figure 9b is a schematic view of a representative embodiment of the transmission gear according to the present invention.

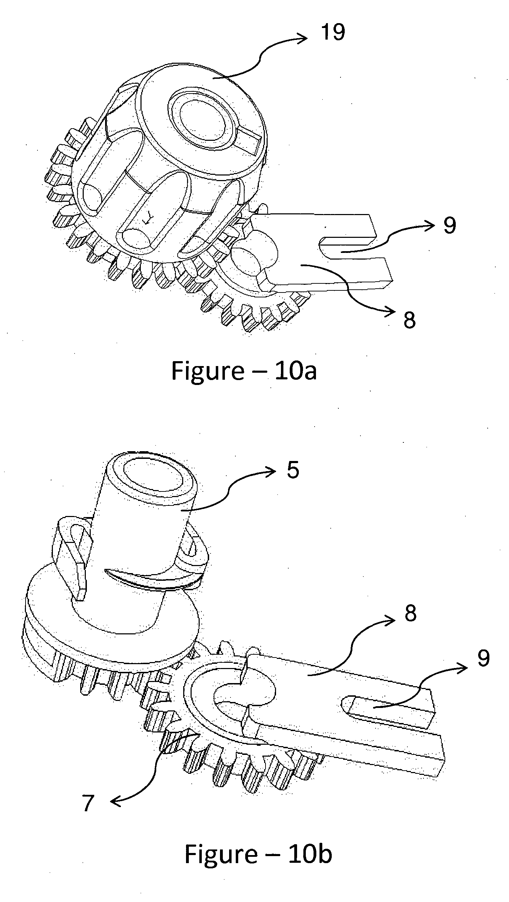

Figure 10a is a schematic view of a representative embodiment of the mechanism according to the present invention.
Figure 10b is a schematic view of a representative embodiment of the mechanism according to the present invention.
Figure 11 a is a schematic view of a representative embodiment of a blister cover winding gears according to the present invention.
Figure 11b is a schematic view of a representative embodiment of a blister cover upper winding gear according to the present invention.
Figure 11c is a schematic view of a representative embodiment of a blister cover middle winding gear according to the present invention.
Figure 11d is a schematic view of a representative embodiment of a blister cover lower winding gear according to the present invention.

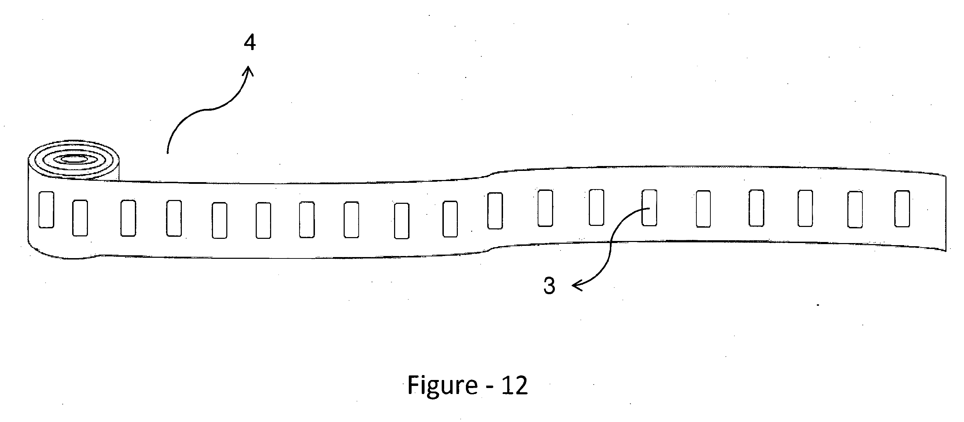

Figure 12 is a schematic view of a representative embodiment of a blister according to the present invention.

### Reference Numbers in Figures

- 1.: Outer body
- 2.: Trigger
- 3.: Cavity
- 4.: Blister
- 5.: Transmission gear
- 6.: Inhaler device
- 7.: Teeth of the actuator lever
- 8.: Actuator lever
- 9.: Groove
- 10.: Connection pin
- 11.: Inner body
- 12.: Lower holder of the inner body
- 13.: Left middle holder of the inner body
- 14.: Right middle holder of the inner body
- 15.: Upper holder of the inner body
- 16.: Upper winding gear of the blister cover
- 17.: Middle winding gear of the blister cover
- 18.: Lower winding gear of the blister cover
- 19.: Guide gear
- 20.: First additional gear
- 21 .: Second additional gear
- 22.: Spring

### Detailed Description of Invention

In the following detailed description, an inhaler device (6) according to the present invention shall be described illustratively by making references to annexed figures, only to make it clear without imposing any restrictions thereon.

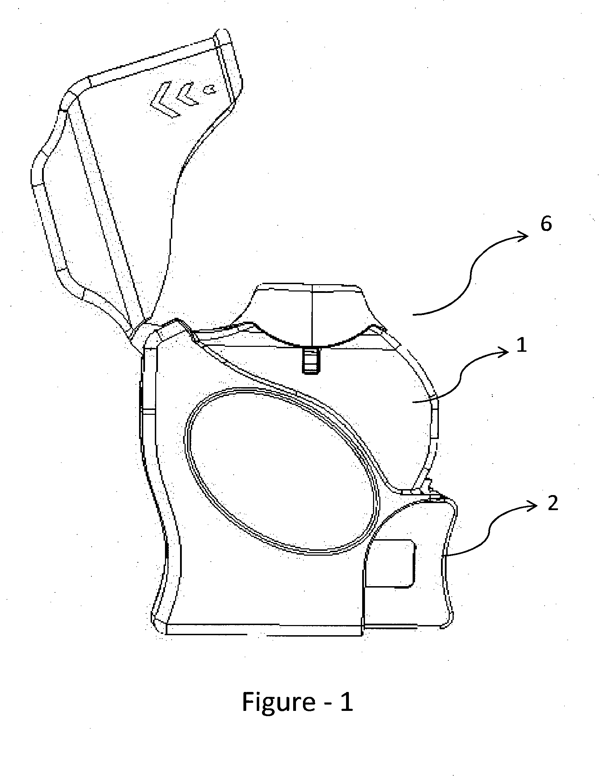

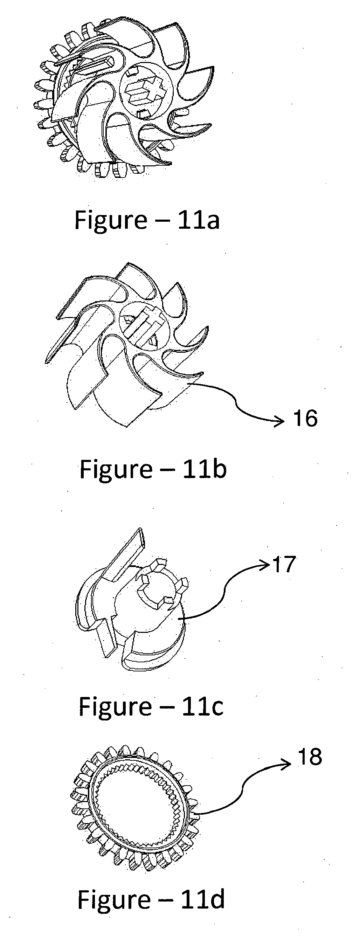

The outer body (1) of the inhaler device (6) according to the present invention as representatively shown in figures 1, 9a, 9b, 11a, 11b, 11c, 11d is obtained by assembling two complementary parts together. The inner periphery of said parts comprises fastening tabs to fasten these parts together, as well as grooves and pins enabling to place the coupled guide gear and transmission gear (19, 5) and the coupled gear set (16, 17, 18) winding the blister cover in the outer body (1). The upper part of the body is provided with a mouthpiece having a drug outlet opening. The lower part of the body is provided with a trigger (2) which can be slid into the body.

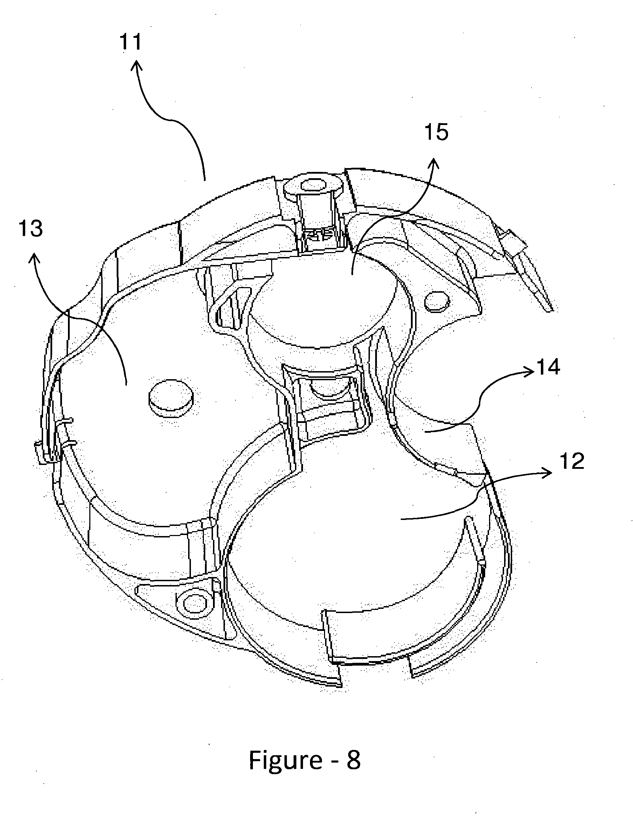

An inner body (11) is disposed in the interior of the outer body (1) to receive a blister, as illustrated representatively in Figure 8. This single-piece body (11) comprises lower, left and right middle and upper holders (12, 13, 14, 15), along with recessed surfaces and fastening tabs. An unused blister (4) is stored in the lower holder (12), this strip-formed blister (4) being extended along intermediate channels and subsequently separated into two parts, i.e. the main layer and the protective layer, by means of the mechanism. The main layer of which the tip is fixed to the second additional gear (21) is held in the left middle holder (13) of the second additional gear (21). The tip of the cover layer, in turn, is fixed to a pin provided on the middle winding gear (17).

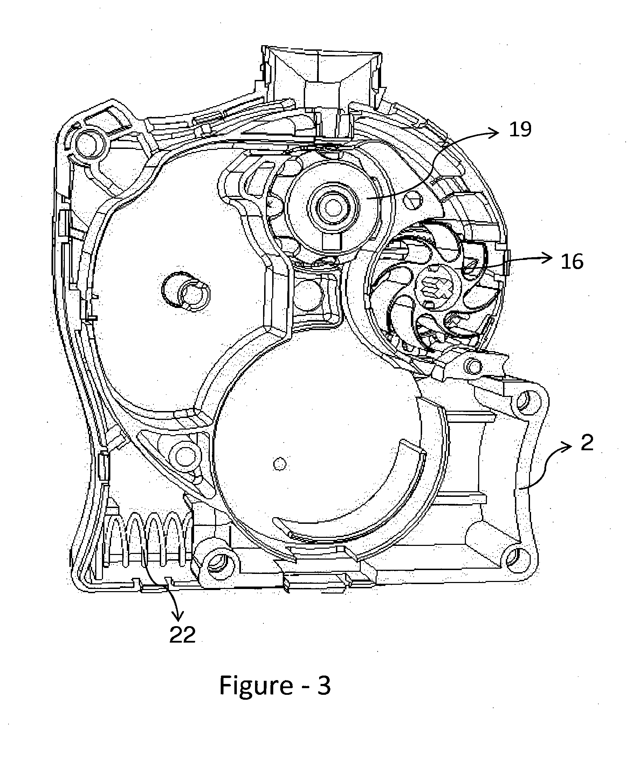

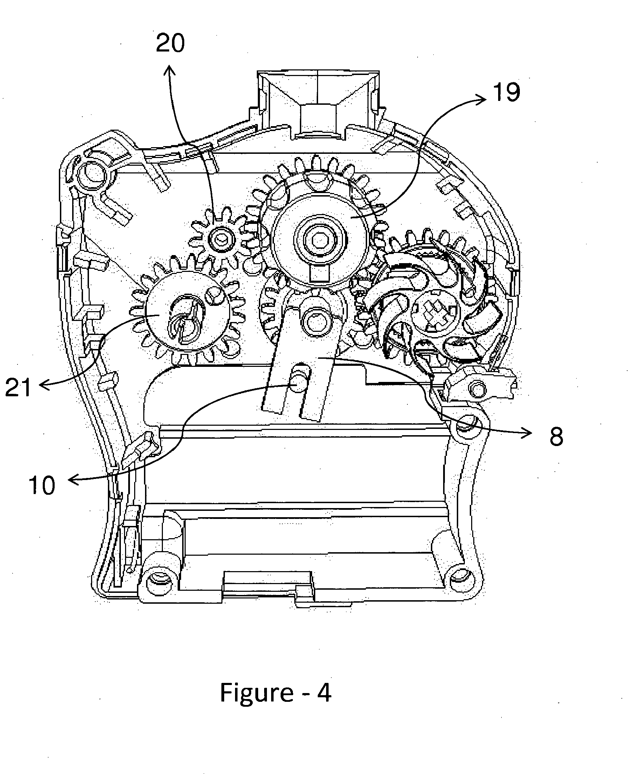

At the beginning of the blister (4) advancement mechanism representatively shown in figures 3 and 4 is provided a trigger (2) slid into a lower part of said outer body (1). The trigger (2) is capable to axially displace in the interior of the body (1). A surface provided on the exterior of the trigger (2) to which a user exerts force to push in the trigger has an inwardly curved structure to provide ease of use. A spring (22) is disposed between the trigger (2) and the inner surface (1) of the outer body, the spring being structured to become compressed (i.e. loaded) when the trigger (2) is pushed into the body (1). The spring (22) is a helical spring.

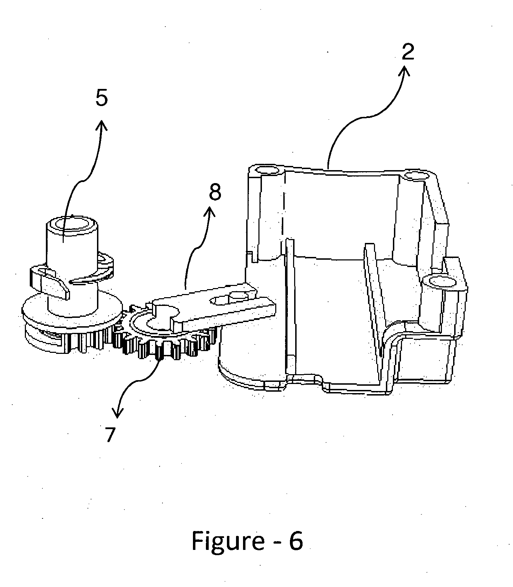

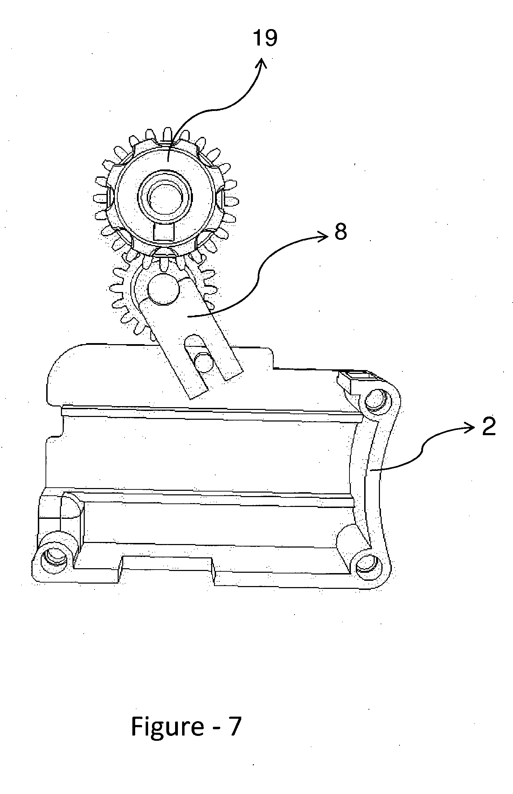

As illustrated in figures 3, 4, 6, 7, an actuator lever (8) is provided, which is coupled to the trigger (2) on one side, and to the circular transmission gear (5) on the other side, and is provided with teeth (7) on at least a portion thereof. This lever (8) comprises a groove (9). On the trigger (2), in turn, is provided a connection pin (10). When this pin (10) is introduced into the groove (9), both a connection is provided, and the actuator lever (8) is enabled to move back and forth and to rotate in a free manner. The actuator lever (8) is either fixed to a whole gear, or a part of the tip of the lever (8) is formed into a circular or linear gear.

When an linear/axial force is exerted to the trigger (2), the actuator lever (8) to which it is coupled to is set in a rotational motion. Thus, an linear/axial motion applied to the trigger is transmitted to the transmission gear (5) as a rotational motion. The rotational motion transmitted to the gear (5) both rotates the transmission gear and the guide gear (19) disposed on the transmission gear, and the lower winding gear of the blister cover (18). In this way, the lower winding gear of the blister cover (18), as well as the upper and middle winding gears of the blister cover (16, 17) are set in rotation. At the same time, both the first additional gear (20) coupled to the guide gear (19) and thus the second additional gear (21) are rotated.

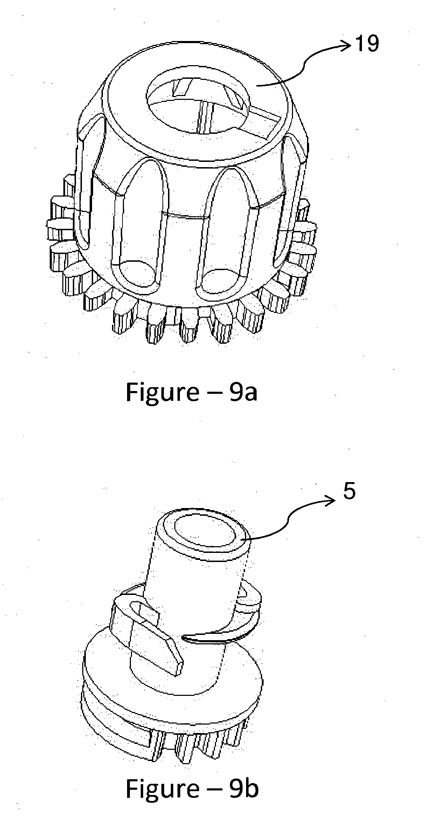

The guide gear (19) of which a representative embodiment is detailed in Figure 9a moves on a single direction and provides only the advancement of the blister. The transmission gear (5) disposed below the guide gear, in turn, is capable to rotate forward and backward.

According to the details given above, the operation of the device according to the present invention is as follows. Following the opening of the lid, a force is exerted by the user to the grip surface of the trigger (2). Then the trigger (2) is driven or slid into the interior of the body (1). The actuator lever (8) which is in connection with the trigger (2) transmits this drive to the transmission gear (5). The transmission gear (5) transmits this drive to the guide gear (19) and to the first and second additional gears (20, 21). Thereafter, the transmission gear and the lower winding gear of the blister lid (18) and the upper and middle winding gears of the blister lid (16, 17) which are disposed on the lower winding gear of the blister lid (18) are set in rotational motion on opposite sides. The winding gears of the blister lid (18, 16, 17) are rotated clockwise, whereas the transmission gear and the guide gear (5, 19) are rotated counterclockwise. The blister (4) in connection with these gears is advanced in the channels of the inner body (11), is passed through and pealed off between the gear sets, and the drug in the cavity is released. The main layer of the blister, now separated into two parts, is rolled in the left middle holder (13), whereas the protective layer is wound onto the upper gear of the blister lid (16).

As a result of sliding the trigger (2) into the interior of the outer body (1), a retaining recess provided on the trigger (2) is coupled to a locking clips lug provided just over itself, resulting in the administration of a single dose of drug. Keeping this slide-in action until the locking position is achieved guarantees the complete peeling-off of the blister and ensures the accurate administration of the required dosage amount. As a result of this locking effect, the trigger (2) becomes retained and it remains out-of-use for a short period of time. This slide-in action also causes the spring (22) to become compressed between the inner surface of the body (1) and the trigger (2).

After the user inhales the powder drug, he/she closes the lid, so that the tip part of the lid exerts force to the rear part of the locking clips, the tip thereof is lifted above, and the clips lug and the retaining recess are detached from each other. As a result of this, the spring (22) in a compressed form pushes back the trigger (2) and restores the device for the next use, without requiring any user intervention (i.e. winding up). However, although the trigger (2) moves backward, the mechanism can not move back the blister. It can be seen from the disclosure above, that the device according to the present invention can be operated by a simple single push action of the user. Thus, when the lid is closed, the device (6) is wound up automatically and is restored for the next use.

According to another embodiment, the actuator lever (8) is fixed to the trigger (2). With this embodiment, sliding the trigger forth and back makes the transmission gear (5) rotate forward and backward. It is further possible to operate the device according to this alternative embodiment.

| **Device type** | **1^{st} trigger push force N with a blister loaded to the device** | **15^{th} trigger push force N with a blister loaded to the device** |
|---|---|---|
| Device according to WO2010114506 and WO2010114505 | 43 | 60 |
| Device according to the present invention | 22 | 25 |

As can be seen in this table, the amount of force which has to be exerted to the trigger of the device by a user to operate the device is reduced to almost half of that was required for the device according to the prior art. The torque generated by means of said arms provides a substantial advantage in the push force required to slide in the trigger.

In result, an inhaler device is obtained with the embodiment disclosed above, wherein this device is extremely accurate, safely operated, the force required to operate the device and exerted to the components of the mechanism is reduced, and wherein cost and size advantages are provided. In place of using a gear between the trigger (2) and the other circular transmission gear (5), an actuator lever (8) is used which is provided with teeth (7) on at least a part thereof to ensure the advantages given above.

The linear motion of the trigger is the sliding in-out motion of the trigger on any direction, including those on the x and y axes. This direction is determined according to the design of the device.

The design of the components used may be varied in alternative embodiments according to the type of the device being produced. In conclusion, the protection scope of the present invention is set forth in the accompanying claims and cannot be restricted to the illustrative disclosures given above, under the detailed description. It is obvious that a person skilled in the relevant art can produce similar embodiments under the light of the foregoing disclosures, without departing from the main principles of the present invention.

## Claims

1. A blister advancement mechanism for a dry powder inhaler device (6) comprising a trigger (2) disposed to axially displace in an outer body of the inhaler device by user exerted force, a transmission gear and a set of interconnected gears for advancement of a blister within the device and, said blister advancement mechanism being structured to advance a blister (4) strip having drug carrying cavities (3) and to release drug from said drug carrying cavities of the blister strip to be released **characterized by** further comprising
- at least one actuator lever (8), being coupled to the trigger (2) on one side by a pivot connection pin (10) of the trigger introduced into a groove (9) of the actuator lever (8), and to the circular transmission gear (5) on the other side, such that the actuator lever (8) is able to perform rotational motion and said actuator lever (8) is provided with teeth (7) on at least a part thereof to transmit a force exerted to the trigger (2) to a gear set and to convert the linear/axial motion of the trigger into circular motion.

2. The blister advancement mechanism according to Claim 1, wherein the teeth (7) are disposed on the actuator lever (8) on a circular surface.

3. The blister advancement mechanism according to any of the preceding claims, wherein the teeth (7) are disposed on the actuator lever (8) on a linear surface.

## Patentansprüche

1. Mechanismus für den Vorschub eines Blisters für eine Vorrichtung (6) zum Inhalieren von Trockenpulver, der einen Auslöser (2) aufweist, der so angeordnet ist, dass er sich in einem äußeren Körper der Inhalationsvorrichtung bei Ausüben einer von einem Benutzer aufgebrachten Kraft axial verschiebt, und ein Übertragungsgetriebe und einen Satz von miteinander verbundenen Zahnrädern für den Vorschub eines Blisters innerhalb der Vorrichtung aufweist, und wobei der Blistervorschubmechanismus so ausgestaltet ist, dass er einen Blister (4) -Streifen, der Medikamente aufnehmende Kavitäten (3) hat, vorbewegt und das Medikament aus den das Medikament aufnehmenden Kavitäten des Blisterstreifens freigibt, **dadurch gekennzeichnet, dass** er weiterhin aufweist:
zumindest einen Betätigungshebel (8), der auf einer Seite mit dem Auslöser (2) durch einen Schwenkzapfen (10) des Auslösers verbunden ist, wobei der Schwenkzapfen in eine Nut (9) des Betätigungshebels (8) eingeführt ist, und der auf der anderen Seite mit einem kreisförmigen Übertragungszahnrad (5) verbunden ist, sodass der Betätigungshebel (8) in der Lage ist, eine Drehbewegung auszuführen und der Betätigungshebel (8) auf zumindest einem Teil desselben mit Zähnen (7) versehen ist, um eine auf den Auslöser (2) ausgeübte Kraft auf einen Getriebesatz zu übertragen und die lineare/axiale Bewegung des Auslösers in eine kreisförmige Bewegung umzusetzen.

2. Blistervorschubmechanismus nach Anspruch 1, wobei die Zähne (7) an dem Betätigungshebel (8) auf einer kreisförmigen Oberfläche angeordnet sind.

3. Blistervorschubmechanismus nach einem der vorstehenden Ansprüche, wobei die Zähne an dem Betätigungshebel (8) auf einer gradlinigen Fläche angeordnet sind.

## Revendications

1. Un mécanisme d'avancement de blister pour un inhalateur de poudre sèche (6) comprenant une gâchette (2) disposée de manière à se déplacer axialement dans un corps extérieur de l'inhalateur sous l'effet de la force exercée par l'utilisateur, un engrenage de transmission et un ensemble de roues dentées interconnectées pour l'avancement d'un blister à l'intérieur de l'inhalateur et ledit mécanisme d'avancement de blister étant structuré pour faire avancer une bande de blister (4) ayant des cavités porteuses de médicament (3) et pour faire sortir le médicament à libérer hors desdites cavités porteuses de médicament de la bande de blister, **caractérisé en ce qu'**il comprend en outre :
- au moins un levier d'actionnement (8) couplé à ladite gâchette (2) d'un côté par une tige de connexion de pivot (10) de la gâchette introduite dans une rainure (9) du levier d'actionnement (8), et audit engrenage de transmission circulaire (5) de l'autre côté, de telle sorte que le levier d'actionnement (8) soit capable d'exécuter un mouvement de rotation et que ledit levier d'actionnement (8) soit au moins partiellement pourvu de dents (7) pour transmettre une force exercée sur la gâchette (2) à un train d'engrenages et pour convertir le mouvement linéaire/axial de la gâchette en un mouvement circulaire.

2. Le mécanisme d'avancement de blister selon la revendication 1, dans lequel les dents (7) sont disposées sur le levier d'actionnement (8) sur une surface circulaire.

3. Le mécanisme d'avancement de blister selon l'une quelconque des revendications précédentes, dans lequel les dents (7) sont disposées sur le levier d'actionnement (8) sur une surface linéaire.
